# FASCICULE DE BREVET EUROPEEN

(11) **EP 0 538 646 B1**
(45) Date de publication et mention de la délivrance du brevet: **23.11.1994**
(21) Numéro de dépôt: 92116505.6
(22) Date de dépôt: 26.09.1992
(51) Int. Cl.: A23C 9/123

(54) **Procédé de préparation d'un lait acidifié**
Verfahren zur Herstellung einer Sauermilch
Process for preparing an acidified milk

(30) Priorité: 25.10.1991 CH 3129/91
(43) Date de publication de la demande: 28.04.1993
(73) Titulaire: SOCIETE DES PRODUITS NESTLE S.A., 1800 Vevey (CH)
(72) Inventeur: Beutler, Ernst, CH-3543 Emmenmatt (CH); Favre-Galliand, Leuka, CH-1005 Lausanne (CH); Illi, Johann, CH-3627 Heimberg (CH); Sutter, Andreas, Yorba Linda, CA 92687 (US)
(74) Mandataire: Wavre, Claude-Alain

(56) Documents cités:
- EP-A- 0 144 274
- DE-A- 1 692 456
- FR-A- 2 073 279
- US-A- 3 897 307
- FOOD TECHNOLOGY. vol. 32, no. 11, Novembre 1978, CHICAGO US page 69 'Dried Yoghurt Product'
- Derwent Publications Ltd., London, GB; AN 90-371973
- RASIC J et al.'Yoghurt, vol.1' 1978, Technical Dairy Publ., Copenhagen DK
- AUSTRALIAN JOURNAL OF DAIRY TECHNOLOGY. vol. 37, no. 4, Décembre 1982, PARKVILLE AU pages 139 - 142 M. BROOME 'The use of cheese whey protein concentrate in the manufacture of skim milk yoghurt'
- Chemical Abstracts 1959, vol.59, no.21, page 20602 c-e (G Chintescu et al.)

## Description

La présente invention a pour objet un procédé de préparation d'un lait acidifié déshydraté ainsi qu'un tel lait obtenu par ce procédé.

Il est connu qu'un lait acidifié tel qu'un yogourt sous forme de gel ou de boissons, par exemple, peut avoir un effet bénéfique pour la santé.

En effet, J. Racic et al. ("yoghurt", 1978, pages 50-52 et 103-104, Tech. Dairy Pub. House, Denmark) présentent les avantages nutritionels que confèrent l'acide lactique L(+) présent dans du yogourt. J. Racic et al. affirment qu'on ne devrait pas nourrir des enfants en bas âges avec un yogourt comprenant de l'acide lactique D(-) car ils ne le métabolisent pas correctement.

Dans cet ordre d'idée, FR 2506129 (EVOG) évoque le fait que, dans la combinaison traditionnelle de microorganismes utilisés pour la fabrication du yogourt, le Lactobacillus bulgaricus se distingue notamment par la formation d'arôme, une acidification rapide et la formation d'acide lactique D(-), alors que le Streptococcus thermophilus se distingue notamment par la formation d'un gel consistant, une acidification lente et la formation d'acide lactique L(+). Ce brevet propose, pour la préparation d'un gel consistant analogue, la souche de Bifidobacterium longum DSM 2054 qui se distingue par la formation d'acide lactique L(+) et qui a été sélectionnée pour son pouvoir acidifiant exceptionnel.

GB 1512890 (YAKULT HONSHA K.K.) propose la préparation d'une boisson lactée acidifiée par fermentation à l'aide d'une combinaison d'une souche de Lactobacillus helveticus capable de produire de l'acide lactique rapidement et d'une souche de Lactobacillus casei faible productrice d'acide, les souches étant sélectionnées de manière que leur combinaison résulte en une acidification rapide sans production de diacétyle ou autre produit secondaire conférant au produit un arôme indésirable.

US 4399220 (Smiley) rappelle, par ailleurs, que le Lactobacillus helveticus, notamment L.helveticus subspecies jugurti est traditionnellement utilisé dans la fabrication de fromages à pâte dure. Ce brevet US 4399220 propose un procédé pour la production d'une culture d'un tel microorganisme dont le pouvoir acidifiant reste particulièrement stable.

Enfin, G. Chintescu et al. (Chemical Abstracts 1959, Vol. 59, page 20602) proposent de préparer un yogourt en poudre par fermentation à l'aide de la combinaison d'une souche de Streptococcus thermophilus et d'une souche de Lactobacillus bulgaricus.

La présente invention a pour but de proposer un procédé de préparation d'un lait acidifié déshydraté qui soit réalisable à l'échelle industrielle, qui permette d'atteindre des pH relativement bas sans provoquer une séparation de protéines et qui fournisse un produit qui se distingue notamment par ses bonnes propriétés de conservation et ses effets favorables sur la santé des consommateurs auxquels il est destiné, notamment les enfants en bas âge et les nourrissons.

A cet effet, dans le procédé de préparation d'un lait acidifié déshydraté selon la présente invention,
- on prépare une solution ou émulsion aqueuse à 10-40% de matière sèche présentant une composition semblable à celle d'un lait,
- on fait fermenter la solution jusqu'à un pH de 4,0-5,0, avec une combinaison d'une ou plusieurs souches de Streptococcus thermophilus et d'une souche de Lactobacillus helveticus formant exclusivement de l'acide lactique L(+), et
- on la sèche jusqu'à moins de 3% en poids d'eau.

On a constaté qu'un tel procédé peut effectivement être réalisé à l'échelle industrielle sans qu'on doive craindre des problèmes de séparation des protéines, malgré le pH relativement bas atteint au cours de l'étape de fermentation. De même, le produit ainsi obtenu présente effectivement de bonnes propriétés de conservation, ses qualités organoleptiques ne subissant aucune modification notable après un entreposage de 12-24 mois à une température de 20-27°C, sous atmosphère de gaz inerte tel que CO₂ et/ou N₂, par exemple, en boîtes en fer étamé laquées fermées hermétiquement, et il est effectivement susceptible d'exercer des effets favorables sur la santé des consommateurs auxquels il est destiné.

Pour mettre en oeuvre le présent procédé, on peut partir de toutes matières premières permettant de réunir des composants dont la somme forme une composition semblable à celle d'un lait tel qu'un lait de vache ou un lait humain.

Dans une première forme de réalisation préférée du présent procédé, on prépare une solution aqueuse à 15-40% en poids de matière sèche présentant une composition semblable à celle d'un lait de vache entier ou écrémé additioné ou non d'une matière grasse lactique et/ou végétale telle que de l'huile de beurre, de l'huile de maïs et/ou de la graisse de coco, par exemple, et d'hydrates de carbone tels que maltodextrine, saccharose, amidon et/ou lactose, par exemple. Pour ce faire, on peut reconstituer un lait de vache en poudre, ou concentrer un lait de vache, à la teneur en matière sèche désirée et on peut lui ajouter ou non de ladite matière grasse et/ou desdits hydrates de carbone.

Une telle solution peut comprendre des protéines composées de 75-82% en poids de caséine et 18-25% en poids de protéines sériques.

De préférence, après avoir fait fermenter cette solution jusqu'à un pH de 4,0-5,0, on la sèche par pulvérisation.

Dans une deuxième forme de réalisation préférée du présent procédé, on prépare une solution aqueuse à 10-40% en poids de matière sèche présentant une composition adaptée aux besoins du nourrisson. Pour ce faire, on peut réunir des composants tels qu'une première partie de sels minéraux, de la caséine acide ou du caséinate de potassium, des protéines sériques, de la crème et/ou de l'huile de maïs et du lait en poudre, notamment, dans des proportions permettant d'approcher la composition d'un lait humain, ajouter facultativement des hydrates de carbone tels que maltodextrine, saccharose, amidon et/ou lactose, par exemple, et reconstituer le tout à la teneur en matière sèche désirée par mélange avec de l'eau adoucie, par exemple. On peut à ce stade homogénéiser la solution, notamment en une ou deux étapes sous 50-200 bar, par exemple.

Une telle solution peut comprendre des protéines composées de 40-82% en poids de caséine et 18-60% en poids de protéines sériques.

De préférence, après avoir fait fermenter cette solution jusqu'à un pH de 4,0-5,0, on concentre la solution fermentée jusqu'à 40-55% en poids de matière sèche par évaporation sous vide, on la supplémente en vitamines et une éventuelle deuxième partie de sels minéraux, et on la sèche par pulvérisation. Cette éventuelle deuxième partie de sels minéraux peut comprendre en particulier des sels de Ca et Mg, que l'on ajoute de préférence après évaporation et soit avant, soit même pendant ou après séchage.

On a constaté qu'il peut être également avantageux d'ajouter de la lécithine soit au cours du mélange initial, soit après évaporation et avant séchage. Enfin, des hydrates de carbone éventuels peuvent être aussi ajoutés après séchage plutôt que lors du mélange initial, notamment dans des régions chaudes où l'on peut craindre des problèmes de collage dans une buse de séchage.

Dans chaque forme préférée de réalisation du présent procédé, pour faire fermenter ladite solution, on lui inocule de préférence 1-5% en volume d'une culture ou d'un mélange de cultures contenant 10⁷-10⁹ germes d'au moins un S.thermophilus et 10⁷-10⁹ germes de L.helveticus par ml et l'on peut incuber durant 2-15 h à 37-45°C.

On peut réaliser cette fermentation en discontinu, à savoir par charges, ou en continu. C'est ainsi que dans une forme de réalisation avantageuse du présent procédé qui se prête particulièrement bien à une exploitation industrielle sur une grande échelle, on réalise la fermentation en continu en deux étapes successives. Dans la première de ces étapes, on peut incuber durant 0,5-5h à pH 5,0-6,0, de préférence pH 5,1-5,7, à 37-45°C. Dans la seconde de ces étapes, on peut incuber durant 1-10h à pH 4,0-5,0 de préférence pH 4,5-4,8, à 37-45°C.

On peut réaliser ces étapes de fermentation dans deux fermenteurs successifs sur une ligne de préparation en continu, le volume de solution traité dans chaque fermenteur étant proportionnel au temps de séjour désiré, par exemple.

Cette forme de réalisation se prête particulièrement bien à un travail optimal en symbiose de chacun des deux microorganismes formant ladite combinaison, la première étape de fermentation favorisant plus particulièrement le travail de la ou des souches de S.thermophilus et la seconde étape favorisant plus particulièrement le travail de la souche de L.helveticus.

Dans chaque forme de réalisation du présent procédé, la ou les souches de S.thermophilus peuvent être choisies parmi les souches vendues dans le commerce pour la préparation de yogourts ou isolées de yogourts, par exemple. La souche de L.helveticus peut être sélectionnée en fonction de son aptitude à former exclusivement de l'acide lactique L(+), parmi les souches vendues dans le commerce pour la préparation de fromage ou lait acidifié ou isolées de fromages ou laits acidifiés, par exemple.

Parmi diverses souches ainsi sélectionnées, trois ont été déposées, à titre d'exemple, selon le Traité de Budapest, le 15.10.91, à la Collection Nationale de Cultures de Microorganismes (CNCM), Institut Pasteur, 28 rue du Dr Roux, 75724 Paris, Cedex 15, France, où elles ont reçu les Nos CNCM I-1154, CNCM I-1155 et CNCM I-1156.

Des détails concernant leur morphologie et leurs propriétés sont donnés ci-après:

### Morphologie

Examinées au microscope ces souches se présentent sous forme de bâtonnets relativement longs et fins.

Ce sont des bactéries Gram-positives microaérophiles qui ne forment pas de spores.

### Métabolisme

Ces bactéries réalisent une fermentation homolactique avec production de lactate comme produit final.
- Différents tests donnent avec chacune d'elles les résultats suivants: activité catalasique (-), production de dioxyde de carbone (-), coagulation du lait (+), hydrolyse de l'arginine (-).
- Elles fermentent les sucres comme indiqué ci-après:

| | CNCM I-1154 | CNCM I-1155 | CNCM I-1156 |
|---|---|---|---|
| L-arabinose | - | - | - |
| D-xylose | - | - | - |
| D-glucose | + | + | + |
| D-mannose | + | - | + |
| D-fructose | + | - | - |
| galactose | + | + | + |
| lactose | + | + | + |
| maltose | - | - | - |
| saccharose | - | - | - |
| trehalose | - | - | - |
| raffinose | - | - | - |
| salicine | - | - | - |
| cellobiose | - | - | - |
| D-sorbitol | - | - | - |
| D-mannitol | - | - | - |
| inositol | - | - | - |
| cellobiose | - | - | - |
| glycerine | - | - | - |
| amidon | - | - | - |

### Milieu de croissance

On peut cultiver ces souches sur milieu MRS ou lait écrémé reconstitué à 10% de matière sèche. La température optimale est de 40-45°C. Le pH après 24 h en lait écrémé reconstitué est de environ 3,40.

### Particularité

Ces trois souches produisent de l'acide lactique L(+) exclusivement (> 99%).

La présente invention a également pour objet le produit obtenu par le présent procédé. Celui-ci est tout particulièrement destiné aux enfants en bas âge et aux nourrissons, sur la santé desquels il peut exercer un effet bénéfique, notamment un effet préventif des diarrhées, en comparaison de produits traditionnels comparables.

Dans une forme de réalisation particulière du présent produit, on prévoit d'y ajouter également une culture lyophilisée de Bifidobacterium.

Les exemples ci-après sont présentés à titre d'illustration du procédé et du produit selon la présente invention. Divers tests sont également présentés à l'appui des qualités du présent produit. Dans ces exemples et tests, les pourcentages sont donnés en poids sauf indication contraire.

### Exemple 1

A un lait de vache entier, on ajoute de l'huile de maïs, du saccharose, de la maltodextrine et de l'amidon, de manière que la solution obtenue présente la composition suivante, en % en poids sur matière sèche:

| | |
|---|---|
| matière grasse du lait | 18,2 |
| huile de maïs | 4,6 |
| protéines du lait (dont environ 80% de caséine et environ 20% de protéines sériques) | 14,5 |
| lactose | 21,2 |
| saccharose | 15,5 |
| maltodextrine | 16,5 |
| amidon | 6,2 |
| minéraux | 3,3 |

On concentre cette solution à 33% de matière sèche par évaporation sous vide.

A cette solution concentrée, on inocule 5% en volume d'une culture contenant, par ml, environ 10⁸ germes d'un Streptococcus thermophilus du commerce et environ 10⁸ germes de la souche Lactobacillus helveticus CNCM I-1154. On fait fermenter la solution concentrée durant environ 12,5 h à 43°C, jusqu'à pH 4,2-4,3. On sèche la solution fermentée par pulvérisation.

On obtient un lait acidifié déshydraté, autrement dit une poudre de lait acidifié, destiné en particulier aux enfants en bas âge et que l'on peut reconstituer à raison de environ 140 g de poudre pour environ 900 ml d'eau. Cette poudre présente une teneur en eau résiduelle de environ 2% et peut se conserver sans altération durant au moins 12 mois à 27°C en boîtes en fer étamé laquées fermées hermétiquement.

### Exemple 2

On procède de manière semblable à celle décrite à l'exemple 1, à l'exception du fait que l'on inocule à ladite solution concentrée la souche de Lactobacillus helveticus CNCM I-1155 au lieu de la souche CNCM I-1154.

On obtient un lait acidifié déshydraté présentant des propriétés comparables à celles du produit obtenu à l'exemple 1.

### Exemple 3

On procède de manière semblable à celle décrite à l'exemple 1, à l'exception du fait que l'on inocule à ladite solution concentrée la souche de Lactobacillus helveticus CNCM I-1156 au lieu de la souche CNCM I-1154.

On obtient un lait acidifié déshydraté présentant des propriétés comparables à celles du produit obtenu à l'exemple 1.

### Test 1

On soumet le lait acidifié déshydraté obtenu à l'exemple 1 à un test de prolifération microbienne en biberon.

A cet effet, on reconstitue ce lait acidifié déshydraté à raison de 139 g de poudre pour 900 ml d'eau et on lui inocule différents microorganismes pathogènes.

Pour comparaison, on inocule les mêmes microorganismes à un lait déshydraté non acidifié, reconstitué, de composition adaptée aux besoins du nourrisson.

Les microorganismes pathogènes utilisés sont les Salmonella typhimurium, Escherichia coli, Staphylococcus aureus et Bacillus cereus. Ils sont inoculés à raison de 10⁴-10⁵ germes par ml de lait reconstitué. Les populations respectives sont dénombrées après 8 h d'incubation à 37°C. Les résultats obtenus sont présentés dans le tableau 1 ci-après:

**Tableau 1**

| Test de prolifération microbienne en biberon. (Inoculation initiale à 10⁴-10⁵ germes/ml - Incubation de 8h à 37°C). | | | | |
|---|---|---|---|---|
| Produit testé | S.typhimurium | E.coli | St.aureus | B.cereus |
| Echantillon de comparaison non acidifié | 4,2.10⁷ | 9,1.10⁷ | 8,0.10⁶ | 8,0.10⁶ |
| Lait déshydraté acidifié selon exemple 1 | < 10 | < 10 | 2,2.10³ | 2,3.10⁴ |

On constate donc que le lait déshydraté acidifé obtenu à l'exemple 1 inhibe complètement la croissance de Staph. aureus et de B.cereus, alors qu'il inactive même S.typhimurium et E.coli.

### Exemple 4

On mélange une première partie de sels minéraux, du caséinate de potassium, des protéines de lactosérum déminéralisé, de la crème, du lait écrémé en poudre, de la maltodextrine et de l'eau adoucie, de manière à obtenir une solution à 20% en poids de matière sèche présentant la composition suivante, en % en poids sur matière sèche:

| | |
|---|---|
| matière grasse du lait | 19,4 |
| huile de maïs (ajoutée à chaud ensuite) | 4,8 |
| lécithine (ajoutée à chaud ensuite) | 0,5 |
| protéines du lait (50% caséine, 50% protéines sériques) | 12,9 |
| lactose | 45,4 |
| maltodextrine | 15,0 |
| minéraux | 2,0 |
| (dont Ca et Mg ajoutés avant, pendant ou après séchage) | |

On préchauffe à 80°C. On ajoute l'huile de maïs. On traite thermiquement par injection de vapeur à 125°C durant 5 s. On homogénéise sous 75 bar à 43°C. On inocule à la solution ou émulsion homogénéisée 2% en volume d'une culture contenant, par ml, environ 10⁸ germes d'un Streptococcus thermophilus du commerce et environ 10⁸ germes de la souche de Lactobacillus helveticus CNCM I-1154.

On fait fermenter la solution durant environ 4,5 h à 43°C jusqu'à pH 4,2-4,3. On refroidit à 10°C.

On préchauffe à 75°C, on concentre par évaporation à 48% de matière sèche. On ajoute la lécithine. On ajoute une deuxième partie de sels minéraux, à savoir des sels de Ca et de Mg et l'on sèche par pulvérisation.

On obtient un lait acidifié déshydraté, autrement dit une poudre de lait acidifié, destiné en particulier aux nourrissons et que l'on peut reconstituer à raison de environ 135 g de poudre pour environ 900 ml d'eau. Cette poudre présente une teneur en eau résiduelle de environ 2% et peut se conserver sans altération durant au moins 12 mois à 27°C en boîtes en fer étamé laquées fermées hermétiquement.

### Exemple 5

On procède de manière semblable à celle décrite à l'exemple 4, à l'exception du fait que l'on inocule à ladite solution ou émulsion la souche de Lactobacillus helveticus CNCM I-1155 au lieu de la souche CNCM I-1154.

On obtient un lait acidifié déshydraté présentant des propriétés comparables à celles du produit obtenu à l'exemple 4.

### Exemple 6

On procède de manière semblable à celle décrite à l'exemple 4, à l'exception du fait que l'on inocule à ladite solution ou émulsion la souche de Lactobacillus helveticus CNCM I-1156 au lieu de la souche CNCM I-1154.

On obtient un lait acidifié déshydraté présentant des propriétés comparables à celles du produit obtenu à l'exemple 4.

### Test 2

On soumet le lait acidifié déshydraté obtenu à l'exemple 4 à un test de prolifération microbienne en biberon.

A cet effet, on reconstitue ce lait acidifié déshydraté à raison de 134 g de poudre pour 900 ml d'eau et on lui inocule différents microorganismes pathogènes de manière semblable à celle décrite au test 1 ci-dessus.

Le tableau 2 ci-après présente les résultats obtenus après une incubation de 8 h à 37°C, en regard des résultats obtenus, pour comparaison, avec un échantillon de lait déshydraté reconstitué de composition semblable, mais non acidifié.

**Tableau 2**

| Test de prolifération microbienne en biberon (Inoculation initiale à 10⁴-10⁵ germes/ml - Incubation de 8 h à 37°C). | | | | |
|---|---|---|---|---|
| Produit testé | S.typhimurium | E.coli | St.aureus | B.cereus |
| Echantillon de comparaison non acidifié | 4,2.10⁷ | 9,1.10⁷ | 8,0.10⁶ | 8,0.10⁶ |
| Lait déshydraté acidifié selon exemple 4 | 10 | 10 | 1,3.10⁴ | 1,9.10⁴ |

On constate donc également que le lait déshydraté acidifié obtenu à l'exemple 4 inhibe complètement la croissance de ces quatre microorganismes pathogènes et qu'il inactive même S.typhimurium et E.coli.

### Test 3

On réalise une étude d'acceptation du lait déshydraté acidifié obtenu à l'exemple 4.

L'étude porte sur 25 nourrissons au cours des trois premiers mois de leur existence. On suit l'évolution de leur taille et de leur poids, et l'on note les régurgitations, la fréquence des selles et leur consistance.

Les courbes de croissance, que les résultats de cette étude permettent d'établir, se situent au beau milieu des domaines considérés comme normaux en Europe et présentent une pente légèrement plus accusée que celle d'une courbe moyenne type.

Les résultats du contrôle des régurgitations et des selles sont regroupés sur le tableau 3 ci-dessous qui présente les quantités moyennes de lait consommées par jour, en ml par kg du nourrisson, valeurs extrêmes entre parenthèses, ainsi que le pourcentage de nourrissons dont le nombre de régurgitations est inférieur à 2 par jour et dont la fréquence et la consistance des selles journalières correspondent aux nombres et aux qualificatifs indiqués.

**Tableau 3**

| Test d'acceptation par des nourrissons du lait déshydraté acidifié selon l'exemple 4. | | | | |
|---|---|---|---|---|
| | | 1er mois | 2ème mois | 3ème mois |
| Consommation de lait (ml/kg) | | 175 (120-243) | 160 (107-203) | 142 (85-181) |
| Régurgitations <2 | | 86 | 94 | 93 |
| Fréquence des selles | < 1 | 4 | 10 | 18 |
| | 1-3 | 83 | 80 | 70 |
| | > 4 | 13 | 10 | 12 |
| consistance des selles | ferme | 63 | 54 | 50 |
| | tendre | 33 | 32 | 35 |
| | liquide | 4 | 14 | 15 |

Les résultats prouvent une excellente tolérance de ce lait acidifié déshydraté par les nourrissons, et une relativement faible tendance à la régurgitation.

### Exemple 7

On mélange en continu une première partie de sels minéraux, du caséinate de potassium, des protéines de lactosérum déminéralisé, de la crème, du lait écrémé en poudre, de la maltodextrine et de l'eau adoucie, de manière à obtenir 4000 litres par heure d'une solution à 20% en poids de matière sèche présentant la composition suivante, en % en poids sur matière sèche:

| | |
|---|---|
| matière grasse du lait | 19,2 |
| huile de maïs (ajoutée à chaud ensuite) | 5,3 |
| lécithine (ajoutée à chaud ensuite) | 0,5 |
| protéines du lait (50% caséine, 50% protéines sériques) | 12,8 |
| lactose | 45,7 |
| maltodextrine | 14,6 |
| minéraux | 1,9 |
| (dont Ca et Mg ajoutés avant, pendant ou après séchage) | |

En continu, on préchauffe la solution à 80°C dans un échangeur de chaleur à plaques. On ajoute l'huile de maïs. On traite thermiquement par injection de vapeur à 125°C durant 5 s. On homogénéise en deux étapes, la première sous 150 bar, la seconde sous 50 bar, à 43°C. On inocule à la solution ou émulsion homogénéisée 1% en volume d'une culture contenant, par ml, environ 5.10⁸ germes de deux Streptococcus thermophilus du commerce et 3% en volume d'une culture contenant, par ml, environ 2.10⁸ germes de la souche de Lactobacillus helveticus CNCM I-1154.

On fait fermenter la solution tout d'abord durant environ 1h à 42°C à pH 5,5 dans un fermenteur de 4000 l, puis durant environ 1,5 h à 42°C à pH 4,7 dans un fermenteur de 6000 l. On préchauffe la solution à 75°C dans un échangeur de chaleur à plaques. On concentre par évaporation à 50% de matière sèche dans un évaporateur à film tombant à trois étages. On ajoute la lécithine. On ajoute une deuxième partie de sels minéraux, à savoir des sels de Ca et de Mg et l'on sèche par pulvérisation.

On obtient en continu un lait acidifié déshydraté, autrement dit une poudre de lait acidifié, destiné en particulier aux nourrissons et que l'on peut reconstituer à raison de environ 134 g de poudre pour environ 900 ml d'eau. Cette poudre présente une teneur en eau résiduelle de environ 2% et peut se conserver sans altération durant au moins 12 mois à 27°C en boîtes en fer étamé laquées fermées hermétiquement.

### Exemple 8

On procède de manière semblable à celle décrite à l'exemple 7, à l'exception du fait que l'on inocule à ladite solution ou émulsion la souche de Lactobacillus helveticus CNCM I-1155 au lieu de la souche CNCM I-1154, et que l'on n'ajoute ladite deuxième partie de sels minéraux qu'après le séchage par pulvérisation.

On obtient un lait acidifié déshydraté présentant des propriétés comparables à celles du produit obtenu à l'exemple 7.

### Exemple 9

On procède de manière semblable à celle décrite à l'exemple 7, à l'exception du fait que l'on inocule à ladite solution ou émulsion la souche de Lactobacillus helveticus CNCM I-1156 au lieu de la souche CNCM I-1154. On obtient un lait acidifié déshydraté présentant des propriétés comparables à celles du produit obtenu à l'exemple 7.

### Exemple 10

Au produit obtenu selon l'un quelconque des exemples 4-9, on ajoute un Bifidobacterium lyophilisé du commerce en quantité telle que la poudre présente une teneur de environ 5.10⁷ germes de Bifidobacterium par g.

## Revendications

1. Procédé de préparation d'un lait acidifié par fermentation avec une culture comprenant un mélange de Streptococcus thermophilus et d'une espèce de Lactobacillus, puis déshydraté, caractérisé en ce que:
- on prépare une solution ou émulsion aqueuse à 10-40% en poids de matière sèche présentant une composition semblable à celle d'un lait,
- on fait fermenter la solution jusqu'à un pH de 4,0-5,0, avec une combinaison d'une ou plusieurs souches de Streptococcus thermophilus et d'une souche de Lactobacillus helveticus formant exclusivement de l'acide lactique L(+), et
- on la sèche jusqu'à moins de 3% en poids d'eau.

2. Procédé selon la revendication 1, dans lequel, pour faire fermenter la solution, on lui inocule 1-5% en volume d'une culture ou d'un mélange de cultures contenant 10⁷-10⁹ germes de S.thermophilus et 10⁷-10⁹ germes de L.helveticus par ml, et l'on incube durant 2-15 h à 37-45°C.

3. Procédé selon la revendication 1, dans lequel, pour faire fermenter la solution en continu, on lui inocule 1-5% en volume d'une culture ou d'un mélange de cultures contenant 10⁷-10⁹ germes de S.thermophilus et 10⁷-10⁹ germes de L.helveticus par ml, et l'on incube à 37-45°C, dans une première étape durant 0,5-5h à pH 5,0-6,0 et dans une seconde étape durant 1-10h à pH 4,2-5,0.

4. Procédé selon la revendication 1, dans lequel ladite souche de L.helveticus est choisie parmi les souches Lactobacillus helveticus CNCM I-1154, CNCM I-1155 et CNCM I-1156.

5. Procédé selon la revendication 1, dans lequel:
- on prépare une solution aqueuse à 15-40% en poids de matière sèche présentant une composition semblable à celle d'un lait de vache entier ou écrémé additionné ou non d'une matière grasse lactique et/ou végétale et d'hydrates de carbone,
- on la fait fermenter jusqu'à un pH de 4,0-5,0, et
- on la sèche par pulvérisation.

6. Procédé selon la revendication 5, dans lequel ladite solution comprend des protéines composées de 75-82% en poids de caséine et 18-25% en poids de protéines sériques.

7. Procédé selon la revendication 1, dans lequel:
- on prépare une solution aqueuse à 10-40% en poids de matière sèche présentant une composition adaptée aux besoins du nourrisson,
- on la fait fermenter jusqu'à un pH de 4,0-5,0,
- on la concentre par évaporation sous vide jusqu'à 40-55% en poids de matière sèche, et
- on la sèche par pulvérisation.

8. Procédé selon la revendication 7, dans lequel on prépare ladite solution aqueuse en mélangeant une première partie de sels minéraux, de la caséine acide ou du caséinate de potassium, des protéines sériques, de la crème, du lait en poudre et de l'eau adoucie, et l'on ajoute une deuxième partie de sels minéraux après la concentration par évaporation sous vide.

9. Procédé selon la revendication 7, dans lequel ladite solution comprend des protéines composées de 40-82% en poids de caséine acide ou caséinate de potassium et 18-60% en poids de protéines sériques.

10. Lait acidifié déshydraté susceptible d'être obtenu par le procédé selon l'une des revendications 1-9, et supplémenté ou non avec une culture lyophilisée de Bifidobacterium.

## Claims

1. A process for the production of a milk acidified by fermentation with a culture comprising a mixture of Streptococcus thermophilus and a lactobacillus species and then dehydrated, in which
- an aqueous solution or emulsion is prepared with a dry matter content of 10 to 40% and a composition similar to that of milk,
- the solution is fermented to a pH of 4.0 to 5.0 with a combination of one or more strains of Streptococcus thermophilus and a strain of Lactobacillus helveticus exclusively forming lactic acid L(+) and
- the solution is dried to a water content of less than 3% by weight.

2. A process as claimed in claim 1, in which the solution is fermented by inoculation with 1 to 5% by volume of a culture or mixture of cultures containing 10⁷-10⁹ germs of S. thermophilus and 10⁷-10⁹ germs of L. helveticus per ml and incubation for 2 to 15 h at 37 to 45°C.

3. A process as claimed in claim 1, in which the solution is continuously fermented by inoculation with 1 to 5% by volume of a culture or mixture of cultures containing 10⁷-10⁹ germs of S. thermophilus and 10⁷-10⁹ germs of L. helveticus per ml and incubation at 37 to 45°C in a first step for 0.5 to 5 h to pH 5.0-6.0 and in a second step from 1 to 10 h to pH 4.2-5.0.

4. A process as claimed in claim 1, in which the L. helveticus strain is selected from the Lactobacillus helveticus strains CNCM I-1154, CNCM I-1155 and CNCM I-1156.

5. A process as claimed in claim 1, in which:
- an aqueous solution is prepared with a dry matter content of 15 to 40% by weight and a composition similar to that of a whole or skimmed cow's milk optionally containing an added lactic and/or vegetable fat and carbohydrates,
- the solution is fermented to pH 4.0-5.0 and
- is dried by spray-drying.

6. A process as claimed in claim 5, in which the solution contains proteins consisting of 75 to 82% by weight of casein and 18 to 25% by weight of whey proteins.

7. A process as claimed in claim 1, in which
- an aqueous solution is prepared with a dry matter content of 10 to 40% by weight and a composition adapted to the needs of babies,
- the solution is fermented to a pH of 4.0 to 5.0,
- concentrated by evaporation *in vacuo* to a dry matter content of 40 to 55% by weight and
- dried by spray-drying.

8. A process as claimed in claim 7, in which the aqueous solution is prepared by mixing a first fraction of mineral salts, acidic casein or potassium caseinate, whey proteins, cream, milk powder and softened water and a second fraction of mineral salts is added after concentration by evaporation in vacuo.

9. A process as claimed in claim 7, in which the solution contains proteins consisting of 40 to 82% by weight of acidic casein or potassium caseinate and 18 to 60% by weight of whey proteins.

10. The dehydrated acidified milk obtained by the process claimed in any of claims 1 to 9 and optionally supplemented with the lyophilized culture of Bifidobacterium.

## Patentansprüche

1. Verfahren zur Herstellung einer durch Fermentation mit einer Kultur, die eine Mischung von Streptococcus thermophilus und einer Lactobacillus-Sorte enthält, angesäuerten und dann dehydratisierten Milch, gekennzeichnet dadurch, daß
- man eine wäßrige Lösung oder Emulsion mit einem Gehalt von 10 bis 40 Gew.-% Trockenmasse herstellt, die eine Zusammensetzung aufweist, die der von Milch ähnlich ist,
- man die Lösung bis zu einem pH von 4,0-5,0 mit einer Kombination von einem oder mehreren Stämmen von Streptococcus thermophilus und einem Stamm von Lactobacillus helveticus fermentiert, die ausschließlich L(+)-Milchsäure bilden,
- und bis zu einem Gehalt von höchstens 3 Gew.-% Wasser trocknet.

2. Verfahren nach Anspruch 1, bei dem man zur Fermentation der Lösung diese mit 1-5 Vol.-% einer Kultur oder einer Mischung von Kulturen beimpft, die 10⁷ - 10⁹ Keime von S.thermophilus und 10⁷ - 10⁹ Keime von L.helveticus pro ml enthält, und man für 2-15h bei 37-45°C inkubiert.

3. Verfahren nach Anspruch 1, bei dem man zur kontinuierlichen Fermentation der Lösung diese mit 1-5 Vol.-% einer Kultur oder einer Mischung von Kulturen beimpft, die 10⁷ - 10⁹ Keime von S.thermophilus und 10⁷ - 10⁹ Keime von L.helveticus pro ml enthält, und man bei 37-45°C während eines ersten Zeitabschnitts von 0,5-5h bei pH 5,0-6,0 und dann während eines zweiten Zeitabschnitts von 1-10h bei pH 4,2-5,0 inkubiert.

4. Verfahren nach Anspruch 1, bei dem der genannte Stamm von L.helveticus ausgewählt ist aus den Stämmen Lactobacillus helveticus CNCM I-1154, CNMC I-1155 und CNMC I-1156.

5. Verfahren nach Anspruch 1, bei dem:
- man eine wäßrige Lösung mit einem Gehalt von 10 bis 40 Gew.-% Trockenmasse herstellt, die eine Zusammensetzung aufweist, die der von Kuh-Vollmilch oder Magermilch, die mit einem Milchfett und/oder Pflanzenfett und Kohlenhydraten ergänzt ist oder nicht, ähnelt,
- man die Lösung bis zu einem pH von 4,0-5,0 fermentiert, und
- durch Sprühtrocknen trocknet.

6. Verfahren nach Anspruch 5, bei dem die genannte Lösung Proteine enthält, die sich aus 75-82 Gew.-% Casein und 18-25 Gew.-% Molkenproteine zusammensetzt.

7. Verfahren nach Anspruch 1, bei dem man:
- eine wäßrige Lösung mit einem Gehalt von 10-40 Gew.-% Trockenmasse herstellt, die eine Zusammensetzung aufweist, die an die Bedürfnisse eines Säuglings angepaßt ist,
- man die Lösung bis zu einem pH von 4,0-5,0 fermentiert,
- durch Eindampfen bei Unterdruck auf einen Gehalt von 40-55 Gew.-% Trockenmasse konzentriert, und
- durch Sprühtrocknen trocknet.

8. Verfahren nach Anspruch 7, bei dem man diese wäßrige Lösung herstellt durch Vermischen eines ersten Teils von Mineralsalzen, saurem Casein oder Kaliumcaseinat, Molkeproteinen, Sahne, Trockenmilch und Süßwasser, und durch Zugabe eines zweiten Teils der Mineralsalze nach dem Konzentrieren durch Eindampfen bei Unterdruck.

9. Verfahren nach Anspruch 7, bei dem die genannte Lösung Proteine enthält, die sich zusammensetzen aus 40-82 Gew.-% saurem Casein oder Kaliumcaseinat und 18-60 Gew.-% Molkeproteine.

10. Trocken-Sauermilch, die erhältlich ist nach dem Verfahren nach einem der Ansprüche 1-9, und die mit einer lyophilisierten Kultur von Bifidobacterium ergänzt ist oder nicht.
